# EUROPÄISCHE PATENTSCHRIFT

(11) **EP 3 586 819 B1**
(45) Veröffentlichungstag und Bekanntmachung des Hinweises auf die Patenterteilung: **23.02.2022**
(21) Anmeldenummer: 19172463.2
(22) Anmeldetag: 03.05.2019
(51) Int. Cl.: A61K 8/36, A61K 8/25, A61Q 19/00, C11D 17/00, C11D 3/12, C11D 3/20, C11D 11/00

(54) **KOSMETISCHE REINIGUNGSPASTE**
COSMETIC CLEANING PASTE
PÂTE DE NETTOYAGE COSMÉTIQUE

(30) Priorität: 21.06.2018 DE 102018210101
(43) Veröffentlichungstag der Anmeldung: 01.01.2020
(73) Patentinhaber: Beiersdorf AG, 20253 Hamburg (DE)
(72) Erfinder: Dorsch, Sabrina, 22337 Hamburg (DE); Peirano, Reto, 22761 Hamburg (DE)
(74) Vertreter: Beiersdorf AG

(56) Entgegenhaltungen:
- DE-A1- 10 221 812
- DE-T2- 69 606 425
- FR-A1- 3 000 382
- US-A1- 2017 181 944

## Beschreibung

Die vorliegende Erfindung betrifft eine kosmetische Suspension in Form einer Paste, die zur Reinigung der Gesichtshaut eingesetzt wird.

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Die tägliche Gesichtsreinigung dient vornehmlich dazu Schweißreste, Fette, abgelagerte Schmutzpartikel und abgestorbene Hautreste effektiv von der Haut zu entfernen. Eine Produktform, die zur Gesichtsreinigung angewendet werden kann, sind Reinigungspasten. Pasten sind Feststoff-Flüssigkeitsgemisch, welche als Suspensionen bezeichnet werden. Derartige Suspensionen enthalten festes partikuläres Material suspendiert in einer Flüssigkeit. Im Gegensatz zu Flüssigkeiten wie reinem Wasser sind Pasten meist Bingham-Fluide, welche erst in einen Fließzustand übergehen, wenn eine bestimmte Schubspannung erreicht wird. Das heißt, dass unter Normalbedingungen die Pasten erst ab einer Mindestschubspannung zu fließen beginnen. Unterhalb davon verhalten sie sich wie ein starrer Festkörper und sind nicht fließfähig. Aufgrund dieses Verhaltens werden Pasten im Gegensatz zu festen Seifenstücken oftmals auch als fest, aber streichfähig beschrieben.

Der Stand der Technik kennt eine Vielzahl an Reinigungspasten für die Haut. Unter anderem sind unter den Mintel GNPD Eintragungsnummern 5145175, 4998631, 4739401 und 3210527 verschiedene Reinigungspasten bekannt, welche entweder zur Reinigung der Hände oder der Gesichtshaut geeignet sind. Jedoch ist zu berücksichtigen, dass Reinigungspasten für die Hände nicht gleichzeitig für die Reinigung des Gesichts geeignet sind. So müssen Reinigungspasten für das Gesicht deutlich milder zur Haut sein. Reinigungspasten für die Hände enthalten hingegen oftmals sehr hohe Anteile von Tensiden und/oder Abrasiva (abrasiv wirkendende Partikeln). Eine Anwendung auf der Gesichtshaut kann somit zu einem schnellen Austrocknen der Gesichtshaut und/oder Hautreizungen führen.

Weiterhin beschreibt WO 03/066797 A1 Reinigungspasten, die jedoch nicht zur Hautreinigung geeignet sind, da sie dazu dienen Flecken aus verunreinigten Textilen zu entfernen.

Ferner kennt der Stand der Technik das Dokument DE 10221812 A1, welches Reinigungszubereitungen mit vorteilhaften taktilen Eigenschaften offenbart. Es werden Suspensionen von Talk in Fettsäuren eingesetzt.

US 2017181944 A1 beschreibt den Zusatz von Talk zur Einstellung einer cremigen Textur in Flüssigseifen.

FR 3000382 A1 offenbart gelförmige Abschminkmittel enthaltend Talk und Fettsäuren. Ferner beschreibt DE 69606425 T2 den Einsatz von Talk zur Reduktion der Austrocknungseigenschaften von Seifen. Jedoch konnte kein Dokument einen Hinweis auf die vorliegende Erfindung geben.

Der Umstand dass der Stand der Technik eine Vielzahl von Produkten kennt, darf jedoch nicht darüber hinwegtäuschen, dass Reinigungsprodukte in Form von Pasten oftmals eine Reihe von Nachteilen aufweisen.

Problematisch ist der Umstand, dass eine Vielzahl von Pasten oftmals klumpig ist. Das heißt, dass bei Entnahme der Paste mit dem Finger, sich die Paste unzureichend cremig anfühlt. So werden taktil beim Verstreichen auf der Haut Verklumpungen in der Paste wahrgenommen. Oftmals sind diese Verklumpungen auch mit den Augen wahrzunehmen. Weiterhin ist es nachteilig, dass sich bei Entnahme der Paste aus dem Tiegel die im Tiegel verbleibende Paste durch die bei der Entnahme entstehende mechanische Belastung leicht in kleinere Bestandteile zersetzt und dementsprechend bröselig ist. Ein weiterer nachteiliger Aspekt von den im Stand der Technik bekannten Pasten ist, dass deren Oberfläche oftmals unzureichend glatt ist, was zur Folge hat, dass die Pasten unzureichend cremig für den Verbraucher wirken.

Aufgabe der vorliegenden Erfindung war es die Nachteile des Standes der Technik zu beseitigen oder die Nachteile des Standes der Technik zu vermindern.

Überraschend wurde gefunden, dass Reinigungspasten gemäß der vorliegenden Erfindung die Nachteile des Standes der Technik nicht aufweisen.

Gegenstand der vorliegenden Erfindung ist eine kosmetische Suspension enthaltend, bezogen auf das Gesamtgewicht der Suspension,
a) 20 Gew.-% bis 40 Gew.-% ein oder mehrere Fettsäuren ausgewählt aus den gesättigten und ungesättigten aliphatischen Monocarbonsäuren enthaltend 8 bis 22 Kohlenstoffatome und/oder deren Alkalisalze; und
b) 1 Gew.-% bis 25 Gew.-% Talk,
dadurch gekennzeichnet, dass die Suspension dadurch erhältlich ist, dass die Fettsäuren und/oder deren Salze aufgeschmolzen und mit weiteren Bestandteilen vermischt werden, wobei das vermischen bei einer Temperatur von mindestens 45°C erfolgt, und anschließend die Abfüllung der erhaltenen Suspension in ein Packmittel bei mindestens 45°C Suspensionstemperatur erfolgt.

Gegenstand der vorliegenden Erfindung ist weiterhin ein Verfahren zur Herstellung einer kosmetischen Suspension gemäß Anspruch 14.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen Suspension. Sollten nachfolgend Verhältnisse von Komponenten/Substanzen/Stoffgruppen offenbart werden, so beziehen diese Verhältnisse auf Gewichtsverhältnisse der genannten Komponenten/Substanzen/Stoffgruppen.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Gemäß der vorliegenden Erfindung enthält die erfindungsgemäße kosmetische Suspension 20 Gew.-% bis 40 Gew.-% ein oder mehrere Fettsäuren und/oder deren Alkalisalze. Erfindungsgemäß werden unter Fettsäuren alle gesättigten und ungesättigten aliphatischen Monocarbonsäuren enthaltend 8 bis 22 Kohlenstoffatome verstanden.

Es ist erfindungsgemäß vorteilhaft, wenn der Gesamtanteil der Fettsäuren und/oder deren Alkalisalze von 22 Gew.-% bis 35 Gew.-% und insbesondere von 24 Gew.-% bis 30 Gew.-% bezogen auf das Gesamtgewicht der Suspension beträgt.

Weiterhin ist es vorteilhaft im Sinne der Erfindung, wenn Palmitinsäure und/oder deren Alkalisalze enthalten sind, wobei es in diesem Fall weiterhin bevorzugt ist, wenn der Anteil an Palmitinsäure und/oder dessen Alkalisalze von 5 Gew.-% bis 15 Gew.-% bezogen auf das Gesamtgewicht der Suspension beträgt.

Weiterhin ist es vorteilhaft im Sinne der Erfindung, wenn Stearinsäure und/oder deren Alkalisalze enthalten sind, wobei es in diesem Fall weiterhin bevorzugt ist, wenn der Anteil an Stearinsäure und/oder dessen Alkalisalze von 5 Gew.-% bis 15 Gew.-% bezogen auf das Gesamtgewicht der Suspension beträgt.

Weiterhin ist es vorteilhaft im Sinne der Erfindung, wenn Myristinsäure und/oder deren Alkalisalze enthalten sind, wobei es in diesem Fall weiterhin bevorzugt ist, wenn der Anteil an Myristinsäure und/oder dessen Alkalisalze von 1,5 Gew.-% bis 5 Gew.-% bezogen auf das Gesamtgewicht der Suspension beträgt.

Weiterhin ist es vorteilhaft im Sinne der Erfindung, wenn Laurinsäure und/oder deren Alkalisalze enthalten sind, wobei es in diesem Fall weiterhin bevorzugt ist, wenn der Anteil an Laurinsäure und/oder dessen Alkalisalze von 1,5 Gew.-% bis 5 Gew.-% bezogen auf das Gesamtgewicht der Suspension beträgt.

Weiterhin ist es vorteilhaft, wenn der Anteil von Ölsäure und/oder deren Alkalisalze weniger als 0,2 Gew.-% bezogen auf das Gesamtgewicht der Suspension beträgt.

Enthält die erfindungsgemäße Zubereitung Arachidinsäure und/oder deren Alkalisalze, so ist es vorteilhaft, wenn der Anteil von Arachidinsäure und/oder dessen Alkalisalze von 0,1 Gew-% bis 0,5 Gew.-% bezogen auf das Gesamtgewicht der Suspension beträgt.

Es ist erfindungsgemäß möglich die Fettsäuren oder deren Alkalisalze in der kosmetischen Suspension einzusetzen. Werden in der kosmetischen Suspension Fettsäuren direkt eingesetzt, so ist es erfindungsgemäß vorteilhaft, wenn zusätzlich eine Base, insbesondere Natriumhydroxid und/oder Kaliumhydroxid zugesetzt werden.

Weiterhin sind vorteilhafte Ausführungsformen der Erfindung dadurch gekennzeichnet, dass diese einen pH-Wert im Bereich von 9 bis 10,5 aufweisen. Vorteilhaft ist damit das Verhältnis von Fettsäuren zu zugesetzten Basen so eingestellt, ist dass diese pH-Werte erhalten werden oder es werden lediglich Alkalisalze der Fettsäuren eingesetzt und die Zugabe von weiteren Säuren oder Basen erfolgt optional, um bei einem abweichenden pH-Wert den pH-Wert in den Bereich von 9 bis 10,5 einzustellen.

Insbesondere vorteilhafte Ausführungsformen der Erfindung enthalten, bezogen auf das Gesamtgewicht der Suspension, 20 Gew.-% bis 40 Gew.-% ein oder mehrere Fettsäuren, 1 Gew.-% bis 25 Gew.-% Talk, wobei die Suspension dadurch gekennzeichnet ist, dass diese einen pH-Wert im Bereich von 9 bis 10,5 aufweist.

Erfindungsgemäß beträgt der Anteil von Talk in der kosmetischen Suspension von 1 Gew.-% bis 25 Gew.-% Talk bezogen auf das Gesamtgewicht der Suspension. Vorteilhaft ist es, wenn der Anteil von Talk in der kosmetischen Suspension von 2,5 Gew.-% bis 10 Gew.-% und insbesondere bevorzugt von 3 Gew.-% bis 7 Gew.-% bezogen auf das Gesamtgewicht der kosmetischen Zubereitung beträgt.

Überraschend hat sich gezeigt, dass die Suspensionen gemäß der Erfindung enthaltend Talk in den vorstehend beschriebenen Anteilen und maximal 30 Gew.-% Fettsäuren die gleiche effektive Reinigungsleistung bei Anwendung auf der Haut zeigen, wie Zubereitungen welche anstatt des Talks höhere Anteile an Fettsäuren, also einen Gesamtfettsäuregehalt von mehr als 30 Gew.-%, insbesondere mehr als 35 Gew.-%, bezogen auf das Gesamtgewicht der Suspension, aufweisen. Dementsprechend war es überraschend möglich, Fettsäureanteile durch Talk zu ersetzen, ohne dass die Reinigungsleistung bei Anwendung auf der Haut reduziert wurde. Da Talk in der Regel ein günstigerer Rohstoff als Fettsäuren ist, konnten auf diese Weise überraschend die Rohstoffkosten für die Herstellung der Suspension reduziert werden, ohne dass die Reinigungsleistung bei Anwendung auf der Haut reduziert wurde.

Weiterhin sind vorteilhafte kosmetische Suspensionen der Erfindung dadurch gekennzeichnet, dass der Anteil von bei 20°C und 1013 hPa flüssigen Bestandteilen mindestens 50 Gew.-% bevorzugt mindestens 55 Gew.-% und insbesondere bevorzugt mindestens 60 Gew.-% bezogen auf das Gesamtgewicht der Suspension beträgt.

Dementsprechend sind vorteilhafte kosmetische Suspensionen dadurch gekennzeichnet, dass diese ein oder mehrere Polyole aufweisend 3 bis 4 Kohlenstoffatome, insbesondere Glycerin und Propylenglykol, enthalten.

Enthält die kosmetische Suspension Glycerin, so ist es vorteilhaft, wenn der Anteil von Glycerin von 15 Gew.-% bis 35 Gew.-%, bevorzugt von 20 Gew.-% bis 30 Gew.-% und insbesondere bevorzugt von 22 Gew.-% bis 28 Gew.-% bezogen auf das Gesamtgewicht der Suspension beträgt.

Enthält die kosmetische Suspension Propylenglykol, so ist es vorteilhaft, wenn der Anteil von Propylenglykol von 1 Gew.-% bis 10 Gew.-%, bevorzugt von 2 Gew.-% bis 8 Gew.-% und insbesondere bevorzugt von 3 Gew.-% bis 7 Gew.-% bezogen auf das Gesamtgewicht der Suspension beträgt.

Weiterhin ist es vorteilhaft, wenn die kosmetische Suspension Wasser in einem Anteil von 20 Gew-% bis 40 Gew.-%, bevorzugt 28 Gew.-% bis 35 Gew.-% bezogen auf das Gesamtgewicht der Suspension enthält.

Vorteilhafte erfindungsgemäße kosmetische Suspensionen enthalten weiterhin Phenoxyethanol. Enthält die kosmetische Suspension Phenoxyethanol, so ist es vorteilhaft, wenn der Anteil von Phenoxyethanol von 0,3 Gew.-% bis 0,8 Gew.-% bezogen auf das Gesamtgewicht der kosmetischen Suspension beträgt.

Weiterhin ist es vorteilhaft, wenn die kosmetische Suspension Ethylparaben enthält, wobei der Anteil von Ethylparaben bevorzugt von 0,1 Gew-% bis 0,4 Gew.-% bezogen auf das Gesamtgewicht der Suspension beträgt.

Weiterhin ist es vorteilhaft, wenn die kosmetische Suspension Methylparaben enthält, wobei der Anteil von Methylparaben bevorzugt von 0,1 Gew-% bis 0,4 Gew.-% bezogen auf das Gesamtgewicht der Suspension beträgt.

Weiterhin ist es vorteilhaft, wenn die kosmetische Suspension Decylene Glycol enthält, wobei der Anteil von Decylene Glycol bevorzugt von 0,1 Gew-% bis 0,8 Gew.-% bezogen auf das Gesamtgewicht der Suspension beträgt.

Weiterhin ist es vorteilhaft, wenn die kosmetische Suspension Caprylyl Glycol enthält, wobei der Anteil von Caprylyl Glycol bevorzugt von 0,1 Gew-% bis 0,8 Gew.-% bezogen auf das Gesamtgewicht der Suspension beträgt.

Weiterhin ist es vorteilhaft, wenn die kosmetische Suspension Ethylhexylglycerin enthält, wobei der Anteil von Ethylhexylglycerin bevorzugt von 0,1 Gew-% bis 0,8 Gew.-% bezogen auf das Gesamtgewicht der Suspension beträgt.

Die kosmetischen Suspensionen der Erfindung zeigen eine überraschend starke Schaumbildung. Das heißt es wird eine große Menge Schaum bei Verreiben der Suspension auf der nassen Haut gebildet. Somit kann überraschend auf den Einsatz von weiteren Tensiden verzichtet werden.

Soll die erhaltene Schaummenge dennoch weiter gesteigert werden, ist es erfindungsgemäß möglich, dass die kosmetische Suspension der Erfindung weitere Tenside enthält.

Als weitere Tenside können erfindungsgemäß insbesondere ein oder mehrere anionische Tenside gemäß der Formel (I)

RO-(CH₂CH₂O)ₓ-(CH₂-CHR¹O)_{y}-(CH₂CH₂O)_{z}-SO₃ M⁺ (I)

enthalten sein, dadurch gekennzeichnet, dass
R ein linearer oder verzweigter C₈-C₁₈-Alkylrest oder Mischungen verschiedener linearer oder verzweigter C₈-C₁₈-Alkylreste ist;
R¹ ein Methyl, Ethyl oder Gemische davon ist;
M⁺ ein Kation, ausgewählt aus der Gruppe bestehend aus Alkalimetallen, NH₄⁺ und HNR² ₃⁺ ist, wobei R² ausgewählt ist aus der Gruppe bestehend aus linearen oder verzweigten Alkylresten, CH₂CH₂OH und CH₂CH(OH)CH₃;
x eine ganzzahlige Zahl im Bereich von 0 - 3 ist;
y eine ganzzahlige Zahl im Bereich 0 - 10 ist; und
z eine ganzzahlige Zahl im Bereich 0 - 30 ist.

Besonders bevorzugte Tenside der Formel (I) sind gewählt aus der Gruppe Sodium Laureth Sulfate (Natriumlaurylethersulfat) und/oder Ammonium Laureth Sulfate (Ammoniumlaurylethersulfat). Enthält die erfindungsgemäße kosmetische Suspension ein anionisches Tensid gemäß der Formel (I), vorzugsweise Sodium Laureth Sulfate (Natriumlaurylethersulfat) und/oder Ammonium Laureth Sulfate (Ammoniumlaurylethersulfat), so ist es bevorzugt, wenn der Anteil dieser anionischen Tenside von 1 Gew.-% bis 10 Gew.-% und bevorzugt von 4 Gew.-% bis 8 Gew.-% bezogen auf das Gesamtgewicht der Suspension beträgt.

Ferner ist es vorteilhaft, wenn die erfindungsgemäße Suspension Sodium Methyl Cocoyl Taurate enthalt, wobei der Anteil von Sodium Methyl Cocoyl Taurate bevorzugt von 0,2 Gew.-% bis 3 Gew.-% bezogen auf das Gesamtgewicht der Suspension beträgt.

Weiterhin enthalten die erfindungsgemäßen Suspensionen vorteilhaft kein Polyethylenglykol.

Vorteilhaft sind die Suspensionen der vorliegenden Erfindung weiterhin dadurch gekennzeichnet, dass diese Bingham Fluide sind. Das heißt die Suspensionen der Erfindung sind vorteilhaft dadurch gekennzeichnet, dass diese erst ab einer Mindestschubspannung zu fließen beginnen, so dass sie sich unterhalb dieser Mindestschubspannung nicht fließfähig sind und erst ab Erreichen der Mindestschubspannung fließfähig werden. Die nötige Mindestschubspannung wird als Fließgrenze der Suspension bezeichnet. Ist die Fließgrenze überschritten, steigt die Schubspannung linear mit der einwirkenden Schergeschwindigkeit an. Derartige Verhalten lassen sich mittels Messung mit einem Rotationsviskosimeter ermitteln. Vorteilhaft weist die erfindungsgemäße Suspension eine Fließgrenze von mindestens 40 Pa, bevorzugt mindestens 60 Pa, weiterhin bevorzugt mindestens 80 Pa, weiterhin bevorzugt mindestens 100 Pa, weiterhin bevorzugt mindestens 120 Pa, weiterhin bevorzugt mindestens 140 Pa und insbesondere bevorzugt von mindestens 160 Pa auf. Die Fließgrenze bezieht sich auf eine Messung mittels dynamischem Oszillationsspannungs-Sweep bei 1 Hz Frequenz und 25ºC mit einem von TA Instruments erhältlichen Rheometers AR2000 unter Verwendung einer 40 mm Durchmesser parallelen Geometrie und einem Spalt von 1000 µm. Für die Ermittlung der Fließgrenze wird das Bingham Modell angewendet.

Ein weiterer überraschender Aspekt der vorliegenden Erfindung betrifft den Umstand, dass, wenn die kosmetische Suspension nach Aufschmelzen der enthaltenen der Fettsäuren und anschließender Mischung der Fettsäuren mit den weiteren Bestandteilen bei einer Mindesttemperatur von 45°C (Temperatur der Suspension) in ein Packmittel abgefüllt wird, die Suspension eine matte gleichmäßige Paste ausbildet. Insbesondere behält die Paste auch bei späterer Entnahme bei Raumtemperatur ihre matte Erscheinung und gleichmäßige Konsistenz bei. Vorteilhaft erfolgt die Abkühlung nach Abfüllung der Suspension bei Raumtemperatur oder kälteren Temperaturen. Bei einer Abfüllung der Suspension bei weniger als 45 °C Suspensionstemperatur zeigt die Suspension eine Art Phasentrennung und erhält nach Anwendung eine ungleichmäßige Konsistenz bzw. die Suspension verflüssigt sich.

Die Vorteile einer erfindungsgemäßen Suspension, die nach dem Zusammenfügen aller enthaltenen Inhaltstoffe bei einer Mindesttemperatur von 45°C (Temperatur der Suspension) in ein Packmittel abgefüllt wurde, sind nachfolgend aufgeführt:
- Die erhaltene Suspension zeigt nach dem Abkühlen weniger Blasen und ist somit gleichmäßiger; und
- die Suspension behält deutlich länger ihre feste, pastöse Konsistenz und verflüssigt sich nicht so schnell, wie eine Suspension, welche auf niedrigere Temperaturen abgefüllt wurden.

Ausführungsformen der Erfindung sind somit dadurch gekennzeichnet, dass die kosmetische Suspension dadurch erhältlich ist, dass diese eine Mindesttemperatur von 45°C (Temperatur der Suspension) beim Abfüllen in ein Packmittel aufweist und vorteilhaft anschließend bei Raumtemperatur oder kälteren Temperaturen auf Raumtemperatur abgekühlt werden. Vorteilhaft beträgt die Raumtemperatur 16°C bis 30°C, insbesondere vorteilhaft 18°C bis 25°C. Eine Abkühlung bei weniger als -20°C ist weiterhin ökonomisch nicht sinnvoll. Somit ist es weiterhin vorteilhaft, wenn das Abkühlen der Suspension nach dem Abfüllen bei einer Temperatur zwischen -20°C bis 30°C erfolgt. Die erfindungsgemäße Suspension ist dadurch erhältlich, dass die Fettsäuren und/oder deren Salze aufgeschmolzen und mit den weiteren Bestandteilen vermischt werden, wobei das vermischen bei einer Temperatur von mindestens 45°C erfolgt, und anschließend die Abfüllung der erhaltenen Suspension in ein Packmittel bei mindestens 45°C Suspensionstemperatur erfolgt. Vorteilhafte Packmittel sind insbesondere Tiegel aus Plastik, aus denen die kosmetische Suspension einfach mit den Fingern entnommen werden kann.

Die kosmetischen Suspensionen gemäß der Erfindung können ferner kosmetische Hilfsstoffe und Wirkstoffe enthalten, wie sie üblicherweise in solchen Zubereitungen verwendet werden, z. B. weitere Wirkstoffe, weitere Konservierungsmittel, weitere Konservierungshelfer, Duftstoffe, Lipide, Farbstoffe und Farbpigmente, Verdickungsmittel, anfeuchtende und/oder feuchthaltende Substanzen oder andere übliche Bestandteile einer kosmetischen oder dermatologischen Formulierung wie weitere Polyole, Schaumstabilisatoren, organische Lösungsmittel oder Silikonderivate, sofern der Zusatz die geforderten Eigenschaften hinsichtlich der Stabilität nicht beeinträchtigen oder ausgeschlossen sind.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Inhaltstoffe** | **Bsp.1** | **Bsp.2** | **Bsp.3** | **Bsp.4** |
|---|---|---|---|---|
| Laurinsäure | 3,4 | 3,4 | 3, 5 | 3,9 |
| Myristinsäure | 3,5 | 4,1 | 3,5 | 12,5 |
| Palmitinsäure | 9,7 | 10,7 | 9,7 | 9,7 |
| Ölsäure | 0,1 | 0,1 | 0,1 | 0,1 |
| Stearinsäure | 9,4 | 10,3 | 9,4 | 9,4 |
| Arachidinsäure | 0,3 | 0,3 | 0,3 | 0,3 |
| Talk | 5 | 10 | 20 | 6 |
| Kaliumhydroxid | 4,8 | 5,5 | 4,8 | 6,8 |
| Glycerin | 25 | 10 | 25 | 25 |
| Propylenglykol | 5 | 5 | 5 | 5 |
| Phenoxyethanol | 0,6 | 0,6 | 0,6 | 0,6 |
| Ethylparaben | 0,3 | | 0,3 | 0,3 |
| Trisodium EDTA | 1 | 1 | 1 | 1 |
| Natriumlaurylethersulfate | | 7 | | |
| Parfum | 0,5 | 0,5 | 0,8 | 0,6 |
| Aqua | ad 100 | ad 100 | ad 100 | ad 100 |

Die Suspensionen der Beispiele Bsp.1 bis Bsp.4 wurden hergestellt, indem die Fettsäuren aufgeschmolzen wurden. Nach mischen mit den weiteren Inhaltstoffen bei 50°C wurden die Suspensionen bei 50°C in einen Tiegel gefüllt und unter Normalbedingungen abgekühlt. Es wurden pastöse Suspensionen erhalten.

In einem weiteren Vergleich zwischen einer ersten Suspension, welche auf bei 50°C abgefüllt wurde, und einer zweiten Suspension, welche bei 40°C abgefüllt wurde, wurde ferner gefunden, dass die erste Suspension eine deutlich geringere Blasenbildung zeigt und deutlich gleichmäßiger ist als die zweite. Die Abfüllung erfolgte in einen Tiegel. Weiterhin wurde gefunden, dass sich die zweite Suspension nach Entnahme von Anteilen der hergestellten Suspension sich schnell verflüssigte, während die erste Suspension ihre Konsistenz behält.

## Patentansprüche

1. Kosmetische Suspension enthaltend, bezogen auf das Gesamtgewicht der Suspension,
a) 20 Gew.-% bis 40 Gew.-% ein oder mehrere Fettsäuren ausgewählt aus den gesättigten und ungesättigten aliphatischen Monocarbonsäuren enthaltend 8 bis 22 Kohlenstoffatome und/oder deren Alkalisalze; und
b) 1 Gew.-% bis 25 Gew.-% Talk,
**dadurch gekennzeichnet, dass** die Suspension dadurch erhältlich ist, dass die Fettsäuren und/oder deren Salze aufgeschmolzen und mit weiteren Bestandteilen vermischt werden, wobei das vermischen bei einer Temperatur von mindestens 45°C erfolgt, und anschließend die Abfüllung der erhaltenen Suspension in ein Packmittel bei mindestens 45°C Suspensionstemperatur erfolgt.

2. Kosmetische Suspension nach Anspruch 1, **dadurch gekennzeichnet, dass** der Gesamtanteil der Fettsäuren und/oder deren Alkalisalze von 22 Gew.-% bis 35 Gew.-% und insbesondere von 24 Gew.-% bis 30 Gew.-% bezogen auf das Gesamtgewicht der Suspension beträgt.

3. Kosmetische Suspension nach einem der vorhergehenden Ansprüche, **dadurch gekennzeichnet, dass** Anteil an Palmitinsäure und/oder dessen Alkalisalze von 5 Gew.-% bis 15 Gew.-% bezogen auf das Gesamtgewicht der Suspension beträgt.

4. Kosmetische Suspension nach einem der vorhergehenden Ansprüche, **dadurch gekennzeichnet, dass** der Anteil an Stearinsäure und/oder dessen Alkalisalze von 5 Gew.-% bis 15 Gew.-% bezogen auf das Gesamtgewicht der Suspension beträgt.

5. Kosmetische Suspension nach einem der vorhergehenden Ansprüche, **dadurch gekennzeichnet, dass** der Anteil an Myristinsäure und/oder dessen Alkalisalze von 1,5 Gew.-% bis 5 Gew.-% bezogen auf das Gesamtgewicht der Suspension beträgt.

6. Kosmetische Suspension nach einem der vorhergehenden Ansprüche, **dadurch gekennzeichnet, dass** der Anteil an Laurinsäure und/oder dessen Alkalisalze von 1,5 Gew.-% bis 5 Gew.-% bezogen auf das Gesamtgewicht der Suspension beträgt.

7. Kosmetische Suspension nach einem der vorhergehenden Ansprüche, **dadurch gekennzeichnet, dass** die Suspension einen pH-Wert im Bereich von 9 bis 10,5 aufweist.

8. Kosmetische Suspension nach einem der vorhergehenden Ansprüche, **dadurch gekennzeichnet, dass** der Anteil von Talk von 2,5 Gew.-% bis 10 Gew.-% und insbesondere bevorzugt von 3 Gew.-% bis 7 Gew.-% bezogen auf das Gesamtgewicht der kosmetischen Zubereitung beträgt.

9. Kosmetische Suspension nach einem der vorhergehenden Ansprüche. **dadurch gekennzeichnet, dass** der Anteil von bei 20°C und 1013 hPa flüssigen Bestandteilen mindestens 50 Gew.-%, bevorzugt mindestens 55 Gew.-% und insbesondere bevorzugt mindestens 60 Gew.-% bezogen auf das Gesamtgewicht der Suspension beträgt.

10. Kosmetische Suspension nach einem der vorhergehenden Ansprüche, **dadurch gekennzeichnet, dass** ein oder mehrere Polyole aufweisend 3 bis 4 Kohlenstoffatome enthalten sind.

11. Kosmetische Suspension nach Anspruch 10, **dadurch gekennzeichnet, dass** Glycerin enthalten ist und der Anteil von Glycerin von 15 Gew.-% bis 35 Gew.-%, bevorzugt von 20 Gew.-% bis 30 Gew.-% und insbesondere bevorzugt von 22 Gew.-% bis 28 Gew.-% bezogen auf das Gesamtgewicht der Suspension beträgt.

12. Kosmetische Suspension nach einem der Ansprüche 10 oder 11, **dadurch gekennzeichnet, dass** Propylenglykol enthalten ist und der Anteil von Propylenglykol von 1 Gew.-% bis 10 Gew.-%, bevorzugt von 2 Gew.-% bis 8 Gew.-% und insbesondere bevorzugt von 3 Gew.-% bis 7 Gew.-% bezogen auf das Gesamtgewicht der Suspension beträgt.

13. Kosmetische Suspension nach einem der vorhergehenden Ansprüche, **dadurch gekennzeichnet, dass** die kosmetische Suspension Wasser in einem Anteil von 20 Gew-% bis 40 Gew.-%, bevorzugt 28 Gew.-% bis 35 Gew.-% bezogen auf das Gesamtgewicht der Suspension enthält.

14. Verfahren zur Herstellung einer kosmetischen Suspension enthaltend, bezogen auf das Gesamtgewicht der Suspension,
a) 20 Gew.-% bis 40 Gew.-% ein oder mehrere Fettsäuren ausgewählt aus den gesättigten und ungesättigten aliphatischen Monocarbonsäuren enthaltend 8 bis 22 Kohlenstoffatome und/oder deren Alkalisalze; und
b) 1 Gew.-% bis 25 Gew.-% Talk,
**dadurch gekennzeichnet, dass** die Fettsäuren und/oder deren Salze aufgeschmolzen und mit weiteren Bestandteilen vermischt werden, wobei das Vermischen bei einer Temperatur von mindestens 45°C erfolgt, und anschließend die Abfüllung der erhaltenen Suspension in ein Packmittel bei mindestens 45°C Suspensionstemperatur erfolgt.

## Claims

1. Cosmetic suspension comprising, based on the total weight of the suspension,
a) 20% by weight to 40% by weight of one or more fatty acids selected from saturated and unsaturated aliphatic monocarboxylic acids comprising 8 to 22 carbon atoms and/or alkali metal salts thereof; and
b) 1% by weight to 25% by weight talc,
**characterized in that** the suspension is obtainable by melting the fatty acids and/or salts thereof and mixing with further constituents, wherein the mixing is carried out at a temperature of at least 45°C, and subsequently filling the resulting suspension into packaging at a suspension temperature of at least 45°C.

2. Cosmetic suspension according to Claim 1, **characterized in that** the total fraction of fatty acids and/or alkali metal salts thereof is from 22% by weight to 35% by weight and particularly from 24% by weight to 30% by weight, based on the total weight of the suspension.

3. Cosmetic suspension according to either of the preceding claims, **characterized in that** the fraction of palmitic acid and/or alkali metal salts thereof is from 5% by weight to 15% by weight, based on the total weight of the suspension.

4. Cosmetic suspension according to any of the preceding claims, **characterized in that** the fraction of stearic acid and/or alkali metal salts thereof is from 5% by weight to 15% by weight, based on the total weight of the suspension.

5. Cosmetic suspension according to any of the preceding claims, **characterized in that** the fraction of myristic acid and/or alkali metal salts thereof is from 1.5% by weight to 5% by weight, based on the total weight of the suspension.

6. Cosmetic suspension according to any of the preceding claims, **characterized in that** the fraction of lauric acid and/or alkali metal salts thereof is from 1.5% by weight to 5% by weight, based on the total weight of the suspension.

7. Cosmetic suspension according to any of the preceding claims, **characterized in that** the suspension has a pH in the range of 9 to 10.5.

8. Cosmetic suspension according to any of the preceding claims, **characterized in that** the fraction of talc is from 2.5% by weight to 10% by weight and particularly preferably from 3% by weight to 7% by weight, based on the total weight of the cosmetic preparation.

9. Cosmetic suspension according to any of the preceding claims, **characterized in that** the proportion of constituents liquid at 20°C and 1013 hPa is at least 50% by weight, preferably at least 55% by weight and particularly preferably at least 60% by weight, based on the total weight of the suspension.

10. Cosmetic suspension according to any of the preceding claims, **characterized in that** one or more polyols having 3 to 4 carbon atoms are present.

11. Cosmetic suspension according to Claim 10, **characterized in that** glycerol is present and the fraction of glycerol is from 15% by weight to 35% by weight, preferably from 20% by weight to 30% by weight and particularly preferably from 22% by weight to 28% by weight, based on the total weight of the suspension.

12. Cosmetic suspension according to either of Claims 10 or 11, **characterized in that** propylene glycol is present and the fraction of propylene glycol is from 1% by weight to 10% by weight, preferably from 2% by weight to 8% by weight and particularly preferably from 3% by weight to 7% by weight, based on the total weight of the suspension.

13. Cosmetic suspension according to any of the preceding claims, **characterized in that** the cosmetic suspension comprises water at a fraction of 20% by weight to 40% by weight, preferably 28% by weight to 35% by weight, based on the total weight of the suspension.

14. Method for preparing a cosmetic suspension comprising, based on the total weight of the suspension,
a) 20% by weight to 40% by weight of one or more fatty acids selected from saturated and unsaturated aliphatic monocarboxylic acids comprising 8 to 22 carbon atoms and/or alkali metal salts thereof; and
b) 1% by weight to 25% by weight talc, **characterized in that** the fatty acids and/or salts thereof are melted and mixed with further constituents, wherein the mixing is carried out at a temperature of at least 45°C, and subsequently the resulting suspension is filled into packaging at a suspension temperature of at least 45°C.

## Revendications

1. Suspension cosmétique contenant, par rapport au poids total de la suspension,
a) 20 % en poids à 40 % en poids d'un ou plusieurs acides gras choisis parmi les acides monocarboxyliques aliphatiques saturés et insaturés contenant 8 à 22 atomes de carbone et/ou leurs sels d'alcali ; et
b) 1 % en poids à 25 % en poids de talc,
**caractérisée en ce que** la suspension peut être obtenue en fondant les acides gras et/ou leurs sels et en les mélangeant avec d'autres ingrédients, le mélange étant réalisé à une température d'au moins 45 °C, et par la suite, le remplissage de la suspension obtenue est réalisé dans un moyen d'emballage à une température de suspension d'au moins 45 °C.

2. suspension cosmétique selon la revendication 1, **caractérisée en ce que** la proportion totale des acides gras et/ou de leurs sels d'alcali est de 22 % en poids à 35 % en poids et en particulier de 24 % en poids à 30 % en poids par rapport au poids total de la suspension.

3. Suspension cosmétique selon l'une quelconque des revendications précédentes, **caractérisée en ce que** la proportion d'acide palmitique et/ou de ses sels d'alcali est de 5 % en poids à 15 % en poids par rapport au poids total de la suspension.

4. Suspension cosmétique selon l'une quelconque des revendications précédentes, **caractérisée en ce que** la proportion d'acide stéarique et/ou de ses sels d'alcali est de 5 % en poids à 15 % en poids par rapport au poids total de la suspension.

5. Suspension cosmétique selon l'une quelconque des revendications précédentes, **caractérisée en ce que** la proportion d'acide myristique et/ou de ses sels d'alcali est de 1,5 % en poids à 5 % en poids par rapport au poids total de la suspension.

6. Suspension cosmétique selon l'une quelconque des revendications précédentes, **caractérisée en ce que** la proportion d'acide laurique et/ou de ses sels d'alcali est de 1,5 % en poids à 5 % en poids par rapport au poids total de la suspension.

7. Suspension cosmétique selon l'une quelconque des revendications précédentes, **caractérisée en ce que** la suspension présente une valeur de pH dans la plage de 9 à 10,5.

8. Suspension cosmétique selon l'une quelconque des revendications précédentes, **caractérisée en ce que** la proportion de talc est de 2,5 % en poids à 10 % en poids et en particulier préférablement de 3 % en poids à 7 % en poids par rapport au poids total de la préparation cosmétique.

9. Suspension cosmétique selon l'une quelconque des revendications précédentes, **caractérisée en ce que** la proportion d'ingrédients liquides à 20 °C et 1 013 hPa est d'au moins 50 % en poids, préférablement d'au moins 55 % en poids et en particulier préférablement d'au moins 60 % en poids par rapport au poids total de la suspension.

10. Suspension cosmétique selon l'une quelconque des revendications précédentes, **caractérisée en ce qu'**un ou plusieurs polyols présentant 3 à 4 atomes de carbone sont contenus.

11. Suspension cosmétique selon la revendication 10, **caractérisée en ce que** de la glycérine est contenue et la proportion de glycérine est de 15 % en poids à 35 % en poids, préférablement de 20 % en poids à 30 % en poids et en particulier préférablement de 22 % en poids à 28 % en poids par rapport au poids total de la suspension.

12. Suspension cosmétique selon l'une quelconque des revendications 10 et 11, **caractérisée en ce que** du propylèneglycol est contenu et la proportion de propylèneglycol est de 1 % en poids à 10 % en poids, préférablement de 2 % en poids à 8 % en poids et en particulier préférablement de 3 % en poids à 7 % en poids par rapport au poids total de la suspension.

13. Suspension cosmétique selon l'une quelconque des revendications précédentes, **caractérisée en ce que** la suspension cosmétique contient de l'eau en une proportion de 20 % en poids à 40 % en poids, préférablement de 28 % en poids à 35 % en poids par rapport au poids total de la suspension.

14. Procédé pour la préparation d'une suspension cosmétique contenant, par rapport au poids total de la suspension,
a) 20 % en poids à 40 % en poids d'un ou plusieurs acides gras choisis parmi les acides monocarboxyliques aliphatiques saturés et insaturés contenant 8 à 22 atomes de carbone et/ou leurs sels d'alcali ; et
b) 1 % en poids à 25 % en poids de talc, **caractérisé en ce que** les acides gras et/ou leurs sels sont fondus et mélangés avec d'autres ingrédients, le mélange étant réalisé à une température d'au moins 45 °C, et par la suite, le remplissage de la suspension obtenue est réalisé dans un moyen d'emballage à une température de suspension d'au moins 45 °C.
